# EUROPEAN PATENT APPLICATION

(11) **EP 4 431 084 A1**
(43) Date of publication of application: **18.09.2024**
(21) Application number: 23382247.7
(22) Date of filing: 16.03.2023
(51) Int. Cl.: A61K 9/08, A61K 9/00, A61K 38/14, A61K 47/26, A61P 31/04

(54) **FORMULATION OF DALBAVANCIN**

(71) Applicant: Adalvo Limited, San Gwann, SGN 3000 (MT)
(72) Inventor: CARRETO CANO, Francisco, Madrid (ES); SANCLIMENS PEREZ DE ROZAS, Gloria, Madrid (ES); STRUSI, Orazio Luca, San Gwann (MT)
(74) Representative: Slavík, Jiri

(57) **Abstract**

The present invention relates to a pharmaceutical formulation containing dalbavancin or a pharmaceutically acceptable salt thereof, in a form of an aqueous solution. The novel formulations have a significantly increased stability over known dalbavancin formulations.

## Description

### Field of Art

The invention relates to a stable and ready to use solution formulation of dalbavancin.

### Background Art

Dalbavancin is a lipoglycopeptide antibiotic. Dalvabancin has strong activity against many Grampositive bacteria, in particular methicillin-sensitive and methicillin-resistant *Staphylococcus aureus, Streptococcus pyogenes, Streptococcus agalactiae, Streptococcus anginosus, Streptococcus intermedius,* and *Streptococcus constellates.*

Dalbavancin (sold under the trade names Dalvance, Xydalba) is used to treat acute bacterial skin and skin structure infections, and it is administered in the form of an infusion solution. Dalbavancin has also been designated by the FDA for the treatment of acute osteomyelitis in children, as an orphan disease.

Xydalba or Dalvance are sold as lyophilized powders which need two steps for preparing the infusion solution, both steps need to be performed aseptically. A first step is a reconstitution. The reconstitution step is carried out by adding water for injections into a vial containing the lyophilized powder and gently swirling or inverting the vial, as there is a danger of foaming. The reconstitution time may be up to 5 minutes. The reconstituted solution must then be further diluted with 5% glucose solution for infusion. It may be challenging to perform the reconstitution step under aseptic conditions in any situation, and the reconstitution time is rather long and burdensome in hospitals. If the reconstitution is not performed correctly and the concentrate starts foaming, it is not suitable for further use.

Moreover, Xydalba or Dalvance is intended to be used immediately and the chemical and physical in-use stability of Xydalba has been demonstrated for both the reconstituted concentrate and for the diluted solution for 48 hours at or below 25 °C.

The formulation of Xydalba or Dalvance contains dalbavancin, mannitol, lactose monohydrate, and hydrochloric acid and sodium hydroxide for pH adjustment. Mannitol acts as stabilizer for dabavancin in the solid phase.

WO 2004/045636, and to some extent also WO 2004/045637, relate to formulations of dalbavancin which correspond to the commercial product. The stabilizers listed in these patent applications include mannitol, lactose, sucrose, sorbitol, glycerol, cellulose, trehalose, maltose, raffinose or mixtures thereof. The present invention aims at providing a formulation of dalbavancin which would be sufficiently stable in liquid form so that the reconstitution step can be avoided.

### Summary of the Invention

The present invention provides a pharmaceutical formulation containing dalbavancin or a pharmaceutically acceptable salt thereof, in a form of an aqueous solution.

It was surprisingly found that an aqueous solution containing a sufficient concentration of dalbavancin or a pharmaceutically acceptable salt thereof is stable. Dalbavancin is a semi-synthetic glycopeptide mixture as described in detail e.g. in EP 1565201 B2.

The pharmaceutically acceptable salt of dalbavancin may be a salt formed by a reaction of an organic acid or an inorganic acid. Such acids may be hydrochloric, hydrobromic, sulfuric, phosphoric, acetic, trifluoroacetic, trichloroacetic, succinic, citric, ascorbic, lactic, maleic, glutamic, camphoric, glutaric, glycolic, phthalic, tartaric, lauric, stearic, salicylic, methanesulfonic, benzenesulfonic, sorbic, picric, benzoic, cinnamic. The pharmaceutically acceptable salt of dalbavancin is preferably dalbavancin hydrochloride.

The pharmaceutically acceptable salt of dalbavancin may alternatively be formed with bases, preferably with alkali metal hydroxides, alkaline earth metal hydroxides, ammonia and amines. For example, the bases may include sodium hydroxide, potassium hydroxide, calcium hydroxide, magnesium hydroxide, baryum hydroxide, ammonia, methylamine, dimethylamine, diethylamine, ethanolamine, picoline.

Water used in the formulation is typically water for injections.

The formulation preferably has a pH within the range of 3 to 5.5, preferably 4 to 5.3, even more preferably about 4.5.

The pH may be adjusted, e.g., by addition of hydrochloride acid and sodium hydroxide.

The reference method for measurement of the content of dalbavancin or a pharmaceutically acceptable salt thereof is HPLC (more specifically, reverse phase HPLC).

The pharmaceutical formulation according to the present invention can be as a concentrate for an infusion solution or parenteral solution. The pharmaceutical formulation according to the present invention can be an infusion solution or a parenteral solution (i.e. ready-to-use solution). The infusion or parenteral solution may be formed from the ready-to-use concentrated solution by dilution with a glucose solution (e.g. 5% (w/v) glucose solution) or with water for infusions.

The concentrate may contain dalbavancin or a pharmaceutically acceptable salt thereof in a concentration of 25 to 100 mg dalbavancin per ml of concentrate, preferably 25 to 70 mg dalbavancin per ml of concentrate, even more preferably 25 to 60 mg dalbavancin per ml of concentrate.

Preferably, the concentration of dalbavancin or a pharmaceutically acceptable salt thereof (re-calculated as dalbavancin) in the aqueous solution (the concentrate) is at least 30 mg/ml, more preferably at least 40 mg/ml, yet more preferably 40 mg/ml to 70 mg/ml, even more preferably 45 to 55 mg/ml. In some embodiments, the concentration of dalbavancin or a pharmaceutically acceptable salt thereof (re-calculated as dalbavancin) in the aqueous solution (the concentrate) is about 50 mg/ml.

The formulation of the invention preferably comprises or essentially consists of dalbavancin or a pharmaceutically acceptable salt thereof, mannitol, lactose monohydrate and water for injection. Preferably, the formulation of the invention is preferably free from any additional stabilizer present in the solution.

The formulation of the invention preferably consists of dalbavancin or a pharmaceutically acceptable salt thereof, mannitol, lactose monohydrate, water for injection and an amount of acid and/or pharmaceutically acceptable base necessary for pH-adjustment. Preferably, the pharmaceutically acceptable acid is hydrochloric acid and/or the pharmaceutically acceptable base is sodium hydroxide.

The formulation of the invention contains at least 95 %, preferably at least 98 %, of the initial amount of dalbavancin or a pharmaceutically acceptable salt thereof after 1 month storage at 5°C ± 3 °C.

The formulation of the invention contains at least 95 %, preferably at least 98 %, of the initial amount of dalbavancin or a pharmaceutically acceptable salt thereof after 1 month storage at 25 °C.

The formulation of the invention contains at least 95 %, preferably at least 97 %, of the initial amount of dalbavancin or a pharmaceutically acceptable salt thereof after 6 months storage at 5°C ± 3 °C.

The formulation of the invention contains at least 95 %, preferably at least 96 %, of the initial amount of dalbavancin or a pharmaceutically acceptable salt thereof after 6 months storage at 25 °C.

The formulation of the invention is stable for more than 48 hours at or below 25 °C, preferably it is stable for more than 1 month at or below 25 °C, more preferably for 3 months at or below 25 °C, even more preferably for 6 months at or below 25 °C.

By "stable", it is understood that it contains at least 90%, preferably at least 95 % of the initial amount of dalbavancin or a pharmaceutically acceptable salt thereof after 48 hours at or below 25 °C.

The formulation of the invention may be administered by infusion or by parenteral injection. It may be administered to prevent a bacterial infection or to treat a bacterial infection. The treatment may be performed once or repeated as needed. Typical delays between individual administrations may range between 1 and 14 days, more preferably between 5 and 9 days.

Bacterial infection may be an infection caused, for example, by Staphylococcus (incl. *Staphylococcus aureus*), *Neisseria, Clostridium, Streptococcus* (incl. *Streptococcus pyogenes, Streptococcus agalactiae, Streptococcus anginosus, Streptococcus intermedius,* and *Streptococcus constellates*) species.

The formulation of the invention may be co-administered with another antibiotic. The co-administration may be sequential or simultaneous. Sequential co-administration refers to administering dalbavancin in another time point than another antibiotic. Simultaneous co-administration refers to administering dalbavancin at the same time point as another antibiotic. In simultaneous co-administration, dalbavancin and another antibiotic may be administered together in one formulation or they may be administered in two (or more) separate formulations, at the same time.

The formulation of the invention may be also used in the treatment of acute osteomyelitis in children.

The formulation of the invention allows to avoid the reconstitution step. This avoids the risk of contamination that may occur during reconstitution. The avoidance of the reconstitution steps ensures a better accuracy of dose. The concentrated aqueous solution requires less time of the hospital personnel to convert to the final form for administration, thus decreasing the need for human resources, and is easier to manipulate for the hospital personnel.

The formulation of the invention is less demanding in terms of time and energy needed for its preparation. The lyophilization process is omitted. The preparation process is thus easier and more straightforward, leading to a ready-to-use product.

### Examples

### Comparative example: Xydalba lyophilized powder

The reference product contains lyophilized powder corresponding to 500 mg dalbavancin. The composition of the lyophilized powder is as follows:

| | |
|---|---|
| Dalbavancin hydrochloride | 515 mg (equivalent to 500 mg dalbavancin) |
| Mannitol | 128.8 mg |
| Lactose monohydrate | 128.8 mg |
| Hydrochloric acid (for pH-adjustment) | q.s. |
| Sodium hydroxide (for pH-adjustment) | q.s. |

The lyophilized powder is reconstituted by addition of 25 ml water, according to the instructions in the Summary of product characteristics of Xydalba. The resulting reconstituted solution has a concentration of 20 mg dalbavancin / 1 ml (25 ml contains 500 mg dalbavancin)

The reconstituted Xydalba solution has been demonstrated to be stable only for 48 hours at or below 25 °C. The summary of product characteristics states that the chemical and physical in-use stability of Xydalba has been demonstrated for both the reconstituted concentrate and for the diluted solution for 48 hours at or below 25 °C. The total in-use stability from reconstitution to administration should not exceed 48 hours. From a microbiological point of view, the product should be used immediately. If not used immediately, in-use storage times and conditions prior to use are the responsibility of the user and would normally not be longer than 24 hours at 2 to 8 °C, unless reconstitution/dilution has taken place in controlled and validated aseptic conditions. Do not freeze.

### Example 1: Composition of the invention - Ready-to-use Dalbavancin concentrated solution

| | |
|---|---|
| Dalbavancin hydrochloride | 52.58 mg/mL |
| | (equivalent to 50.95 mg/mL of dalbanvancin base) |
| Mannitol | 12.89 mg/mL |
| Lactose monohydrate | 12.89 mg/mL |
| Water for injection | q.s. 1.00 mL |
| Hydrochloric acid (for pH-adjustment) | q.s. pH 4.5 |
| Sodium hydroxide (for pH-adjustment) | q.s. pH 4.5 |

The formulation was prepared by the following procedure:
80% of the total volume of water was poured into the beaker. Lactose monohydrate was added and the beaker was shaken until complete dissolution. Mannitol was added and the beaker was shaken until complete dissolution. Dalbavancin hydrochloride was added gently, avoiding the formation of foam. The pH value was adjusted by addition of NaOH or HCl solution as needed. The solution was made up to reach the weight according to density (1.026 g/mL) by addition of water. The product was shaken for 10 minutes.

The concentration of dalbavancin is 50 mg / 1 ml. The pH of the solution is 4.5.

### Example 2: Stability testing assay

In this example, the dalbavancin content (%) at the start (T0), and after 1 month (T1) are measured. Vials containing 5 ml of formulation prepared according to example 1 was stored at temperatures described below over a period of 1 month (T1).
5°C ± 3°C (conditions for storage in a refrigerator)
25°C ± 2°C / 60% RH ± 5% RH (long term stability study conditions)
40°C ± 2°C / 75% RH ± 5% RH (accelerated stability study conditions)

Freshly prepared formulation and formulation stored for 1 month were then tested for dalbavancin content. The dalbavancin content was determined using HPLC. The HPLC column used was Ultimate XB-C18 (4.6 mm x 250 mm x 5 µm). Sample solvent was water : acetonitrile (70:30 v/v), column temperature 50 °C, flow rate 1.0 mL/min. 10 microliters of sample were injected on each run. Mobile phase was A: 0.025 mol/L aqueous sodium dihydrogen phosphate : acetonitrile (90:10), B: 0.025 mol/L aqueous sodium dihydrogen phosphate : acetonitrile (30:70). Gradient of mobile phase was from 67%A and 33%B to 30%A and 70%B, and then back to 67%A and 33%B. Detection was performed by UV/VIS detector at 280 nm.

The table in example 2 shows the relative dalbavancin content in the stored formulation, relative to the nominal amount of dalbavancin used for preparation of the formulation, expressed as a percentage.

| **Preparation** | **T0** | | | **T1** | | |
|---|---|---|---|---|---|---|
| | 5°C | 25°C 60% RH | 40°C 75% RH | 5°C | 25°C 60% RH | 40°C 75% RH |
| Example 1 | 102.2 % | 102.2 % | 102.2 % | 101.6 % | 101.4 % | 91.6 % |

### Example 3: Stability testing (impurities)

In this example, the total impurities content (%) at the start (T0), and after 1 month (T1) under the conditions indicated in the table (temperature, relative humidity) was measured.

Vials containing 5 ml of formulation prepared according to example 1 was stored at temperatures described below over a period of 1 month (T1).
5°C ± 3°C (conditions for storage in a refrigerator)
25°C ± 2°C / 60% RH ± 5% RH (long term stability study conditions)
40°C ± 2°C / 75% RH ± 5% RH (accelerated stability study conditions)

Freshly prepared formulation and formulation stored for 1 month was then tested for the content of impurities (related substances). The content of impurities was determined using HPLC. The HPLC column used was Phenomenex Aeris Peptide XB-C18 (4.6 mm x 250 mm x 3.6 µm) and Waters Xbridge Peptide BEH C18 (4.6X150mm, 3.5 µm), combined in series. Sample solvent was water : acetonitrile (70:30 v/v), column temperature 50 °C, flow rate 1.0 mL/min. 10 microliters of sample were injected on each run. Mobile phase was A: 50mM aqueous ammonium dihydrogen phosphate (pH 5.5): acetonitrile (95:5), B: 50mM aqueous ammonium dihydrogen phosphate (pH 5.5) : acetonitrile (40:60). Gradient of mobile phase was from 70%A and 30%B to 40%A and 60%B, and then back to 70%A and 30%B. Detection was performed by UV/VIS detector at 220 nm.

The total amount of impurities is shown as weight percentage, relative to the nominal amount of dalbavancin used for preparation of the formulation.

| **Preparation** | **T0** | | | **T1** | | |
|---|---|---|---|---|---|---|
| | 5 °C | 25 °C 60% RH | 40 °C 75% RH | 5 °C | 25 °C 60% RH | 40 °C 75% RH |
| Example 1 | 1.0 | 1.0 | 1.0 | 0.7 | 1.0 | 4.2 |

### Example 4: Product compositions

**Composition of 500 mg dalbavancin concentrated solution**

| | |
|---|---|
| Dalbavancin hydrochloride | 515 mg (equivalent to 500 mg of dalbanvancin base) |
| Mannitol | 125 mg |
| Lactose monohydrate | 125 mg |
| Water for injection | 10 ml |
| Hydrochloric acid (for pH-adjustment) | q.s. pH 4.5 |
| Sodium hydroxide (for pH-adjustment) | q.s. pH 4.5 |

**Composition of 1000 mg dalbavancin concentrated solution**

| | |
|---|---|
| Dalbavancin hydrochloride | 1030 mg (eq. to 1000 mg dalbavancin) |
| Mannitol | 250 mg |
| Lactose monohydrate | 250 mg |
| Water for injection | 20 ml |
| Hydrochloric acid (for pH-adjustment) | q.s. pH 4.5 |
| Sodium hydroxide (for pH-adjustment) | q.s. pH 4.5 |

**Composition of 1500 mg dalbavancin concentrated solution**

| | |
|---|---|
| Dalbavancin hydrochloride | 1545 mg (eq. to 1500 mg dalbavancin) |
| Mannitol | 375 mg |
| Lactose monohydrate | 375 mg |
| Water for injection | 30 ml |
| Hydrochloric acid (for pH-adjustment) | q.s. pH 4.5 |
| Sodium hydroxide (for pH-adjustment) | q.s. pH 4.5 |

The resulting formulation of all these product compositions is a ready to use product, there is no reconstitution step needed. The concentration of dalbavancin is 50 mg / 1 ml. The pH of the solution is 4.5.

The present invention allows to produce vials with higher amounts of dalbavancin. The doses typically administered to patients are typically 500 mg or 1000 mg or 1500 mg. With the products currently available on the market, the higher doses require the use of 2 or 3 vials of lyophilized powder, each vial requiring a separate reconstitution procedure. With the products of the invention, the dose of dalbavancin is contained in only one vial, and only one dilution step is performed with a single vial, instead of two or three separate reconstitution and dilution steps required to be carried out separately for each of the three vials.

## Claims

1. A pharmaceutical formulation comprising dalbavancin or a pharmaceutically acceptable salt thereof, in a form of an aqueous solution wherein the concentration of dalbavancin or a pharmaceutically acceptable salt thereof is above 25 mg dalbavancin per ml.

2. The formulation according to claim 1, comprising and/or essentially consisting of dalbavancin or a pharmaceutically acceptable salt, mannitol, lactose monohydrate and water for injection.

3. The formulation according to claim 2, which is free from any additional stabilizer present in the solution.

4. The formulation according to any one of the preceding claims, consisting of dalbavancin or a pharmaceutically acceptable salt, mannitol, lactose monohydrate, water for injection and a pharmaceutically acceptable acid and/or a pharmaceutically acceptable base in amounts necessary for pH-adjustment.

5. The formulation according to any one of the preceding claims, wherein the concentration of dalbavancin or a pharmaceutically acceptable salt thereof is from 25 to 100 mg dalbavancin per ml.

6. The formulation according to any one of the preceding claims, wherein the concentration of dalbavancin or a pharmaceutically acceptable salt thereof is from 45 to 55 mg dalbavancin per ml.

7. The formulation according to any one of the preceding claims, which contains at least 95 % of the initial amount of dalbavancin or a pharmaceutically acceptable salt thereof after 1 month storage at 5°C ± 3 °C.

8. The formulation according to any one of the preceding claims, which contains at least 95 % of the initial amount of dalbavancin or a pharmaceutically acceptable salt thereof after 1 month storage at 25 °C.

9. The formulation according to any one of the preceding claims, which contains at least 95 % of the initial amount of dalbavancin or a pharmaceutically acceptable salt thereof after 48 hours at or below 25 °C.

10. The formulation according to any one of the preceding claims, which contains at least 95 % of the initial amount of dalbavancin or a pharmaceutically acceptable salt thereof after 1 month at or below 25 °C.

11. The formulation according to any one of the preceding claims for use in prevention a bacterial infection or in the treatment of a bacterial infection, in particular in the prevention or treatment of an infection caused by *Staphylococcus, Neisseria, Clostridium,* or *Streptococcus* species.

12. The formulation for use according to claim 11, wherein the formulation is co-administered simultaneously or sequentially with another antibiotic.

13. The formulation according to any one of the claims 1 to 10 for use in the treatment of acute osteomyelitis in children.
